# EUROPEAN PATENT APPLICATION

(11) **EP 1 790 716 A1**
(43) Date of publication of application: **30.05.2007**
(21) Application number: 05077683.0
(22) Date of filing: 23.11.2005
(51) Int. Cl.: C12N 5/12, C12N 5/10, C07K 14/705, C07K 16/00, A61K 39/395

(54) **Uses of the FcRn receptor**

(71) Applicant: UMC Utrecht Holding B.V., 3584 CM Utrecht (NL)
(72) Inventor: Vidarsson, Gestur, 3602 XC Maarssen (NL); van de Winkel, Johannes Gerardus Joseph, 3707 CK Zeist (NL)
(74) Representative: Winckels, Johannes Hubertus F.

(57) **Abstract**

The invention relates to the field of biochemistry and molecular biology. More specifically the invention relates to the production of (monoclonal) antibodies and even more specifically to the production of therapeutic and diagnostic antibodies. The invention provides a method for producing an antibody or a functional part, derivative and/or analogue thereof in a cell, comprising providing a cell that is capable of producing said antibody with FcRn receptor protein and culturing said cell to allow for production of said antibody.

### Annex to the application documents - subsequently filed sequences listing

SEQUENCE LISTING

<110>  UMC Utrecht Holding B.V.

<120>  Uses of the FcRn receptor

<130>  P74482EP00

<140>  EP05077683
<141>  2005-11-23

<160>  3

<170>  PatentIn version 3.3

<210>  1
<211>  20
<212>  DNA
<213>  artificial

<220>
<223>  primer

<400>  1
ctcagggtgg agctggaatc                                                    20


<210>  2
<211>  20
<212>  DNA
<213>  artificial

<220>
<223>  primer

<400>  2
ctccacgaag ggagatccaa                                                    20


<210>  3
<211>  28
<212>  DNA
<213>  artificial

<220>
<223>  probe

<400>  3
atcgtcatcg gtgtcttgct actcacgg                                           28

## Description

The invention relates to the field of biochemistry and molecular biology. More specifically the invention relates to the production of (for example monoclonal) antibodies and derivatives thereof and even more specifically to the production of therapeutic and diagnostic antibodies and derivatives thereof.

Immunotherapy using monoclonal antibodies (mAb) has emerged as an important therapeutic strategy for cancer, immune deficiencies, immune regulation and autoimmunity (reviewed in ^{1,2}). An important advantage of antibody therapy is the ability to target causative disease agents with great selectivity. Furthermore, therapeutic antibodies can interact with immune cells and activate complement, which contributes significantly to their therapeutic efficacy. The technique to generate monoclonal antibodies was developed in 1975 by Drs. Köhler and Milstein ³, who were awarded the Nobel Prize for this finding in 1984. The first therapeutic monoclonal antibody (OKT3) was approved in 1986 by the US Food and Drug Administration (FDA) to reverse acute kidney transplant rejection. This antibody originated from mice and induced strong immune responses in humans, which limited the use of this drug, as it could only be used safely for a short period of time. To circumvent this problem, chimeric (70% human) and humanized (~80-95% human) antibodies were produced. Later techniques have allowed for generation of synthetic human antibodies by Phage Display technology, which are more compatible for use in humans. With the development of genetically engineered human immunoglobulin-transgenic mice, it is now possible to generate fully human antibodies, which enable repeated administration for more effective therapy in humans ^{1,2}.

The first US antibody that was FDA-approved for tumor treatment, the chimeric antibody Rituximab, is directed to the CD20 target on B cells, and was shown to be very effective in the treatment of non-Hodgkin's lymphoma. Antibody therapy has also proved successful against solid tumors, as demonstrated by Trastuzumab, directed against Her-2/neu (c-erbB2), which is overexpressed in ~30% of breast cancers. Antibody therapy is also used against infectious agents in the clinic (Synagis, directed against Respiratory Syncytial Virus), against rejection after organ transplantation (Zenapax, and Simulect), and against autoimmune diseases like Crohn's disease and rheumatoid arthritis (the TNF-alpha specific antibody Remicade) ^{1,2}. Currently, 18 antibodies have been approved by the US FDA for clinical use (Table 1) ⁴, and more than 400 antibodies are currently in development for clinical use.

A drawback of this promising new line of therapeutics is the necessity of repeated administration of high amounts used per patient (up to grams per patient), combined with the high manufacturing costs (relative to small molecules). The high treatment costs limit the widespread use of therapeutic antibodies, and raises ethical questions on their use in select patient groups. New techniques and methods that can shorten production time, and/or increase production yields are expected to make antibody drugs more cost-effective and more readily available for clinical use.

The goal of the present invention is to provide methods and means to increase production yield and/or quality and/or reduce production time and/or reduce production costs. The invention discloses, amongst others, that FcRn receptor protein and/or increased expression thereof, promotes higher antibody concentrations produced in the fluid surrounding antibody-producing cells. Without being bound by theory it is believed that this is at least in part due to increased secretion of antibody by an antibody-producing cell.

Thus in a first embodiment, the invention provides a method for producing an antibody or a functional part, derivative and/or analogue thereof in a cell, comprising providing a cell that is capable of producing said antibody with FcRn receptor protein and culturing said cell to allow for production of said antibody.

In respect of the term "producing" it is noted that the above-described steps cannot only be used for the production of an antibody but said steps can also be used in a method for increasing the production of an antibody. In the last case said cell does already produce (small amounts of) antibody and the production is increased by introducing or increasing the amount of FcRn receptor protein expressed by said cell.

An antibody (or immunoglobulin; the terms are used interchangeably herein) is typically described as a protein of which the monomer is a "Y"-shaped molecule that consists of two identical heavy chains and two identical light chains. The chains are connected via disulfide bonds. Each heavy chain has a constant region, which is the same in all immunoglobulins of the same class, and a variable region which differs between immunoglobulins of different B-cells, but is the same in all immunoglobulins of the same B cell. Depending on the specific antibody isotype, the heavy chain has a constant region composed of three or four domains. The variable part domain of any heavy chain is composed of one domain. Each light chain has two domains: one constant and one variable domain.

An antibody is composed of two heavy and two light chains and said antibody can be divided in two Fab (fragment antigen binding) fragments and one Fc (fragment crystallizable) fragment. The Fc fragment is the stem of the "Y" and is composed of two heavy chains that each contributes two or three constant domains, depending on the class of antibody.

Although a method according to the invention is, amongst others, based on the observation that FcRn promotes IgG secretion by IgG-producing cells, the invention is not limited to (natural) IgG antibodies, because production of chimeric antibodies, antibody derivatives, antibody equivalents, antibody analogues and antibody fragments is state of the art. Examples of said chimerics, derivatives, equivalents, analogues and fragments will be discussed in more detail below.

Hence, the invention also provides a method for producing or a method for increasing production of a functional part, derivative and/or analogue of an antibody. Functional in this context means: capable of binding to FcRn receptor protein in a manner simulating the properties of IgG-FcRn binding. According to current knowledge, the Fc of IgG (as well as albumin) binds to FcRn receptor protein. Moreover, it is known that the interface between CH2 and CH3 of Fc interacts with FcRn receptor protein ⁵⁻⁷. Antibody chimerics, derivatives, equivalents, analogues and fragments comprising the Fc of IgG or comprising at least the CH2-CH3 interface of Fc are therefore also included herein.

An example of a functional part of an antibody is an IgG molecule which essentially comprises at least one antigen binding site, as well as an FcRn binding part, i.e. essentially all non-antigen binding parts and all non-FcRn binding parts have been removed. An example of a derivative of an antibody is a chimeric molecule consisting of a receptor ligand binding domain (i.e. CD40-Ligand) and the FcRn binding part of an IgG antibody (CD40L-Fc), i.e. a so-called IgG half-molecule. An example of an analogue is for example the murine analogue of a certain human IgG antibody (or part or derivative thereof) or another molecule which binds to FcRn in a manner simulating IgG: high/medium affinity binding at pH 6.5 and lower, but no or low binding at physiological pH 7.4 (i.e. albumin). All parts, derivatives and/or analogues thereof must at least comprise an FcRn binding part, for example the complete Fc fragment of an IgG antibody or a functional part thereof, i.e. a part that is capable of binding to FcRn (in kind not necessarily in amount), for example the CH2-CH3 interface of Fc.

The presently claimed invention is thus not only useful in the production of for example a monoclonal antibody, but can also be used in the production of bispecific antibodies, Small Modular Immuno-Pharmaceuticals (SMIPs), all variations of the monoclonal technology like quadroma's and / or oligoclonics, and all molecules with FcRn-binding characteristics similar to IgG (binding at low pH, no or low binding at physiological pH of above 7). It may also be used by exchanging or modifying the extracellular binding domain of FcRn, but retaining the intracellular tail and function of FcRn, to accommodate for enhanced production of novel molecules. Depending on the production method currently used for these molecules, one can for example manipulate the cells producing said molecules to also produce FcRn or to produce more/additional FcRn. The production of said molecules is then enhanced by the presence of said FcRn.

A class of IgG receptor is the neonatal FcγR (FcRn) receptor, which consists of a unique α-chain and β2-Microglobulin (β2M), and represents a major histocompatibility class I (MHC-I) homolog ⁸, which has been found in epithelial cells, placental syncytiotrophoblasts, as well as endothelial cells. In these cells FcRn has been implicated in transport of IgG across mucosal cells ^{8,9}, from mother to foetus ¹⁰, and regulation of IgG half-life ¹¹⁻¹⁴, respectively. In these tissues, FcRn binds and transports IgG, providing newborns with humoral immunity, and prolonging IgG lifetime. Recently we identified FcRn to be expressed on phagocytes, where it functions together with 'classical' leukocyte FcγR in mediating internalization (phagocytosis) of IgG-coated targets ¹⁵. Recent findings indicate FcRn to be instrumental in the transport of albumin ¹⁶, and to mediate transport of IgG to mucosal surfaces, and mucosal antigen sampling 9.

The invention not only comprises the use of an FcRn receptor protein but also of a functional part, derivative and/or analogue thereof. In this context the term functional means that said part, derivative and/or analogue must still be capable of binding to an Fc fragment of an IgG molecule (or to albumin), preferably having the same or a comparable /similar pH dependency. Even more preferred said functional part, derivative and/or analogue of FcRn also comprises all the elements necessary for trafficking between the lysosome and the cell membrane, for example the appropriate signal sequences and recircularisation signals. The crystal structure of FcRn is known ^{5,17,18} and it is furthermore reported that the crystal structure of human and rat FcRn are similar ^{5,7}. Moreover, also the Fc binding site is very homologous ^{5,7,18} and hence a skilled person has all the necessary information to develop, without undue burden, a functional part, derivative and/or analogue of FcRn. One nonlimiting way of testing the functionality of a functional part, derivative and/or analogue of an FcRn receptor protein is by testing its binding capacity to, for example Fc or albumin, at pH 6.5 and 7.4. Around pH 6.5 binding between said functional part, derivative and/or analogue and its said Fc or albumin should occur, but not at a pH of around 7.4.

The cells in a method according to the invention are allowed sufficient time to grow or live in a cell culture medium (i.e. the cells are cultured or incubated under appropriate conditions which may vary depending on the specific cell used) to allow for production of an antibody. The presence of FcRn receptor protein promotes antibody secretion by an antibody-producing cell and hence antibody production is provided for and/or improved.

Optionally, the produced antibody is harvested and/or purified by techniques known by a skilled person, such as by protein A or Protein G affinity purification according to the manufacturer's instructions (Amersham, Uppsala, Sweden).

Depending on the required amount of antibody, a method according to the invention is scaled up from a small, to medium or large-scale. Different scales involve different culture systems ranging from T-flask (small), roller bottles, Cell Line (Integra Biosciences, Chur, Switzerland), Erlenmeyer flasks (medium) to larger (custom built) fermentor systems (large).

The cell that is used in a method according to the invention, i.e. a cell that is capable of producing an antibody, can be obtained/derived in various ways. One can for example use a cell that is capable by its nature of producing antibodies, for example a B-cell, or a B-cell derived cell (NS-0, SP2/0), or a hybridoma cell. However, one can also start with a cell which under natural conditions does not produce antibodies but which cell is (genetically) manipulated to produce an antibody, for example Chinese Hamster Ovary cells (CHO), Baby Hamster Kidney cells, 293 cells and derivatives thereof or other adenovirus E1 expressing cell lines like PER.C6. The requirement of such a cell is that it is a eukaryotic cell, capable of providing a functional environment for FcRn. By "functional environment" is meant that FcRn has to be functional in the cell (capable of transport), which means that the cell has to express the necessary molecular machinery. Preferably such a cell is provided with a nucleic acid that encodes an antibody or a functional part, derivative and/or analogue thereof. Even more preferred, said nucleic acid is functionally linked to a regulatory sequence, such as a promoter sequence and an enhancer sequence. In a preferred embodiment said cell is part of a cell line, preferably an immortal or immortalized cell line. In yet another preferred embodiment, such a cell is further provided with FcRn. As will be explained in more detail below, in another preferred embodiment, the invention provides a, for example human, cell in which the endogenous FcRn promoter has been replaced with a strong (constitutive) promoter or with an inducible promoter or a cell in which the endogenous FcRn promoter has been induced.

The FcRn protein receptor molecule is provided as a protein or as a nucleic acid encoding said FcRn receptor protein (or a functional part, derivative and/or analogue thereof). If FcRn receptor protein is provided as a protein it is preferably delivered with a protein transfection reagent (e.g. Chariot, Active Motif, Carlsbad, CA) or coupled to a transport moiety/protein transduction peptide which enables entry of a fusion of said moiety/peptide and said FcRn into a cell. An example of such a moiety/peptide is the HIV-TAT protein. However, to establish a more stable situation it is preferred that said FcRn receptor protein is provided as a nucleic acid. In a preferred embodiment, the invention provides a method for producing an antibody or a functional part, derivative and/or analogue thereof in a cell, comprising providing a cell that is capable of producing said antibody with FcRn receptor protein and culturing or incubating said cell to allow for production of said antibody, wherein said cell is provided with a nucleic acid encoding said FcRn receptor protein. Although it is possible to maintain the nucleic acid encoding FcRn protein receptor as an extrachromosomal entity it is preferred that said nucleic acid is incorporated in the genome of the cell. This is for example accomplished by homologous recombination between the nucleic acid encoding FcRn and the chromosome of the used cell (line). Preferably recombination is performed in a targeted manner to ensure that the nucleic acid encoding FcRn is incorporated in a transcriptionally active part of the chromosome. It is clear to the skilled person that said nucleic acid encoding FcRn is functionally linked to regulatory sequences, for example a promoter and optionally to an enhancer sequence.

In a preferred embodiment, FcRn receptor protein is overproduced in the used cell. Overexpression is for example accomplished by providing multiple copies of FcRn functionally coupled to regulatory sequences and/or by providing a limited amount of copies (for example one or two) that are linked to strong regulatory promoters (e.g. Cytomegalovirus, (CMV) promoter or modifications thereof) or a combination thereof.

It is clear to the skilled person that besides providing a cell with FcRn receptor protein as a protein or as a nucleic acid encoding said FcRn receptor protein (or a functional part, derivative and/or analogue thereof) it is furthermore possible to, for example, induce or increase expression of FcRn receptor protein in a cell that does already comprise the genetic information in respect to FcRn. This is for example accomplished by switching on, for example by adding an inducer to said cell, the promoter in front of the FcRn (α-chain) gene and/or the promoter in front of the β2-microglobulin gene. In yet another embodiment, FcRn receptor protein is provided by, for example, replacement of the endogenous promoter in front of the α-chain with a strong promoter or an inducible promoter.

In case one would like to produce or increase the production of a human antibody in a human cell in the presence of a human FcRn receptor protein, induction of the endogenous FcRn promoter or replacement of the endogenous with a strong or inducible promoter, is accomplished by using standard (and well known) molecular biology techniques.

In yet another preferred embodiment, the invention provides a method for producing an antibody or a functional part, derivative and/or analogue thereof in a cell, comprising providing a cell that is capable of producing said antibody with FcRn receptor protein and culturing said cell to allow for production of said antibody, wherein said antibody is derived from a first species and said cell is derived from the same or a different species. An example of the latter case is a human antibody produced in a mouse or in murine cells.

It is clear to the skilled person that multiple combinations of the introduced α-chain of FcRn and of the β2-microglubulin of FcRn with the FcRn chains optionally already present in said cell can be made. In a preferred embodiment, the invention provides a method for producing an antibody or a functional part, derivative and/or analogue thereof in a cell, comprising providing a cell that is capable of producing said antibody with FcRn receptor protein and culturing said cell to allow for production of said antibody, wherein at least the α-chain of FcRn receptor protein is provided or over-produced. Such a method is especially useful if β2-microglobulin expression of the cell is not a limiting factor for said cell. In yet another embodiment, said method further comprises providing or over-producing β2-microglobulin, where β2-microglobulin (quality / quantity) is limiting. It is clear that which particular parts of FcRn are introduced, largely depend on the cell type used. Normally a cell will comprise enough β2M and hence introduction of β2M will not be necessary. If the cell does not or does not comprise sufficient β2M this must also be introduced. In a preferred embodiment, said α-chain and/or said β2-microglobulin is/are derived from the same species. In an even more preferred embodiment, the used cell is also derived from the same species, i.e. if the cell is of human origin, said cell is preferably provided with or over-produces a human α-chain of FcRn and optionally also a human β2-microglubulin. In yet an even more preferred embodiment, the invention provides a method for producing an antibody, wherein antibody produced by said cell is derived from the same species as said FcRn receptor α-chain, or derived from a functional analogue of said species. In the described example this would mean that the antibody is also human. An example of a functional analogue of said species is an antibody derived from the same genus. In yet another preferred embodiment, the α-chain and/or the β2M as well as the antibody are derived from the same species and the cell is derived from another species. In a preferred example the α-chain and/or the β2M are derived from a human as well as the antibody and the used cell is rodent cell, preferably a chinese hamster or murine cell.

As already explained above, one of the important applications of a method according to the invention is the production of human antibodies that are used as a therapeutic compound and hence in a preferred embodiment, the produced antibody is derived from a human. Even more preferably, said human antibody is produced in a human cell and in the presence of a human FcRn. In this case the endogenous promoter in front of FcRn is induced or the promoter in front of FcRn is replaced with a strong (constitutive) promoter or with an inducible promoter. Another important application is in the field of diagnostic antibodies where the produced antibody is preferably a mouse antibody and wherein said antibody is produced in a mouse.

It is clear to the skilled person that different kinds of cells can be used, for example cells derived from different species (human, murine, etc.). Moreover, said cells may or may not by nature be capable of producing antibodies. It is clear that the easiest way to generate a high producing IgG cells is by providing a cell, that is naturally capable of producing antibodies, with a nucleic acid that encodes an antibody, for example a B-cell derived cell (as mentioned above), because such a cell is already provided with the required molecular machinery for antibody production, which may not (or not sufficiently) be the case for cells that do not naturally produce antibodies. In a preferred embodiment, the invention provides a method for producing an antibody or a functional part, derivative and/or analogue thereof in a cell, comprising providing a cell that is capable of producing said antibody with FcRn receptor protein and culturing said cell to allow for production of said antibody, wherein said cell is derived from a human or rodent. A preferred rodent is a Chinese hamster or a mouse. An example of a suitable mouse cell is a hybridoma derived from a normal mouse that produces mouse IgG or from a transgenic mouse that produces human IgG.

The cell that is used in a method may not only by present in a collection of cells (i.e. present in a cell culture) but may also be part of a non-human animal. Examples of a suitable non-human animal are a cow, a goat, a mouse, a rat, a rabbit, a pig or a sheep and so on. Moreover, also chickens and their eggs are a suitable production platform. The choice for the selection of a specific transgenic system for the production of an antibody (or one of the above described derivatives thereof) depends on several factors and include genetic traits, the size of the animal, the volume of milk or blood, ease of generation, feasibility of tissue-specific expression, quality of the produced antibody (stability, functionality, half-life). A skilled person can, based on public available information on these factors, easily select the most suitable non-human animal.

A transgenic non-human animal can be produced in a variety ways, amongst others via pronuclear microinjection and nuclear transfer. The method to obtain a transgenic animal is preferably performed in such a way that site-specific gene transfer is obtained. Transchromosomal non-human animals are also included herein.

Preferably such a non-human animal is provided with a nucleic acid encoding the desired antibody (or a functional part, derivative and/or analogue thereof) and in a more preferred embodiment said animal is also provided with a nucleic acid encoding FcRn ((or a functional part, derivative and/or analogue thereof). If for example mouse A comprises a nucleic acid encoding an antibody and mouse B comprises a nucleic acid encoding FcRn, a mouse comprising both nucleic acid can, for example, be obtained by breeding mouse A and B.

In yet another preferred embodiment, the invention thus provides a non-human animal comprising a nucleic acid encoding FcRn (or a functional part, derivative and/or analogue thereof). In a preferred embodiment, said nucleic acid is heterologous if compared to naturally present nucleic acid sequences in said animal, for example a nucleic acid sequence encoding the human α-chain of FcRn in a mouse. In yet another preferred embodiment, the invention provides a non-human animal comprising a replacement in respect of the FcRn promoter, for example the endogenous FcRn promoter is replaced with a strong (constitutive) promoter or with an inducible promoter. In another preferred embodiment, the invention provides a non-human animal comprising an induced endogenous FcRn promoter.

In yet another embodiment, the invention provides a plant comprising a nucleic acid encoding FcRn (or a functional part, derivative and/or analogue thereof). Preferably, such a transgenic plant comprises a nucleic acid encoding the α-chain of FcRn as well as a nucleic acid encoding β2M. Even more preferably, such a plant is also provided with a nucleic acid encoding an antibody (or any of the mentioned derivatives thereof).

In yet another embodiment, a collection of different antibody producing cells is used in a method according to the invention, resulting in the production of a mixture of antibodies (polyclonal mixture or omniclonal mixture, i.e. multiple antibodies produced by a single, clonal cell line). In a preferred embodiment, a method according to the invention is started with a cell that produces a single type of antibodies and hence such a method results in the production of a monoclonal antibody, for example a murine monoclonal antibody or a human monoclonal antibody or a functional fragment, derivative and/or analogue thereof.

In yet another embodiment, human analogues are used in any of the methods of the invention, for example an antibody derived from a primate or a α-chain and/or β2-microglobulin derived from a primate. It is clear that the invention can also be used in the production of primate or primatized antibodies.

In a preferred embodiment, the produced antibody is IgG. As with β2M and the α-chain of FcRn, both the heavy and light chains of immunoglobulins are synthesized and secreted into endoplasmic reticulum, where they assemble into fully functional heterodimers in the case of FcRn, or a dimer of heterodimers for IgG (2 heavy and two light chains). As already outlined above, IgG-half molecules as well as IgG derivatives and / or analogues thereof are also included herein as well as bispecific antibodies, SMIPs, quadroma's (omniclonal lines, same light chain but different H chain), provided that they bind to FcRn, or modifications thereof, in a pH-dependent manner as described above, and the modified FcRn retains normal intracellular routing mechanism of FcRn.

Different kinds of cells, suitable for use in a method according to the invention, have already been discussed above and two more, general cell types, will be discussed here. In a preferred embodiment, the invention provides a method for producing an antibody or a functional part, derivative and/or analogue thereof in a cell, comprising providing a cell that is capable of producing said antibody with FcRn receptor protein and culturing said cell to allow for production of said antibody, wherein said cell is a hybridoma or a transfectoma.

A hybridoma is a fusion between a B-cell and a myeloma tumor cell. Such a hybridoma has the advantage that the resulting cell line is immortal and hence production of a monoclonal antibody can be essentially guaranteed throughout time. The generation of IgG producing hybridoma cells involves both *in vitro* or *in vivo* phases. First a mouse is immunized with a target antigen. Subsequently B-cells (normally from the spleen or lymph nodes) are fused with a "fusion partner" (e.g. NS-1 or SP2/0 cells) for immortalization. Subsequently, the resulting cells are selected on a single-cell level and characterized by their ability to secrete antibodies to the target antigen. FcRn can be provided before or after said fusion. An already established hybridoma cell line is provided with FcRn after the cell fusion, but not yet fused cells can also be provided with FcRn at the time of fusion with the myeloma tumor cell (e.g. the fusion partner has already been provided with FcRn).

A transfectoma is, in the present context, a cell which has been provided with a nucleic acid encoding an antibody or a functional part, derivative and/or analogue thereof. An example of such a transfectoma is a CHO, BHK or NS-0 cell that has been provided with a nucleic acid encoding an antibody or a functional part, derivative and/or analogue thereof. FcRn can be provided before, at the same time or after transfection of the cell with the nucleic acid encoding said antibody or a functional part, derivative and/or analogue thereof.

In a preferred embodiment the nucleic acid encoding said antibody or a functional part, derivative and/or analogue thereof is coupled to regulatory sequences, such as a promoter and optionally also enhancer sequences.

Antibody production systems can be based on *in vitro* or *in vivo* systems. It is clear that *in vitro* strategies can be easily maintained and propagated. However, *in vivo* systems have the advantages that they can be induced to increase the yield of the harvested IgG. The invention thus provides a method for producing and antibody or for increasing the production of an antibody which method is an in vivo system (for example ascites fluid produced in a mouse) or an *in vitro* system (for example an immortal cell line). As already discussed above an in vivo system is preferably performed with help of a transgenic non-human animal which comprises a(n) (additional) (heterologous) nucleic acid encoding FcRn. Examples of a suitable non-human animal are a cow, a mouse, a rat, a pig, a chicken, a rabbit or a goat.

The invention further provides a cell capable of producing antibodies which cell has been provided with FcRn or with at least one additional copy of a nucleic acid encoding FcRn. The presence of such an FcRn molecule (or its nucleic acid) is easily determined with help of standard biochemistry and/or molecular biology techniques. Even more preferably the invention provides a non-human animal with at least one (additional) copy of a nucleic acid encoding (for example human) FcRn. Examples of such an animal is a mouse or a hamster. Such a mouse is for example obtained by crossing a mouse comprising a nucleic acid encoding human IgG with a α chain FcRn knockout mouse (and preferably a double knockout mouse also lacking functional mouse β2-microglobulin) that has been provided with a nucleic acid encoding human FcRn. The resultant mice comprise a nucleic acid encoding human IgG as well as a nucleic acid encoding human FcRn and are believed to be high human IgG expressing animals. In a preferred embodiment, the FcRn receptor protein and the antibody are derived from one and the same species (for example human) and the animal is derived from another species (for example mouse). In another preferred embodiment, the FcRn receptor protein comprises a modified version from one species more compatible to function with IgG of another species (for example human) and the animal is derived from same species as FcRn (for example mouse).

The invention furthermore provides use of FcRn receptor protein (or a functional part, derivative and/or analogue thereof) for providing (or increasing) expression of an antibody (or a functional part, derivative and/or analogue thereof) in a cell.

FcRn receptor protein (or a functional part, derivative and/or analogue thereof) may be provided as a protein or as a nucleic acid encoding said receptor protein.

If the cell in question does already contain the information for producing the desired antibody (or a functional part, derivative and/or analogue thereof), a nucleic acid encoding said antibody need not be provided. In case the cell does not contain said information, said cell is further provided with a nucleic acid encoding said antibody.

Antibody-producing cells (such as B cells), like other cells, take up fluid phase components by pinocytosis. In endothelial cells (and other FcRn expressing cells), most proteins taken up by this process are degraded. IgG molecules, however, are rescued from lysosomal degradation by FcRn, and recycled back to the surrounding media ^{11,12}. FcRn-mediated IgG recycling is saturable in *vivo* ^{4,12}, thus not only secretion of IgG by IgG-producing cells may be dependent on FcRn, but also its recycling. To experimentally address this, we performed competition studies, in which we cultured equal numbers of human 293 cells (which express endogenous FcRn) with no, or increasing amounts of exogenous polyclonal human IgG. Exogenously added IgG was shown to inhibit IgG secretion from these cells, in accordance with the saturable nature of IgG-recycling by FcRn. These data show that production of IgG by cells is subject to (a) negative feed-back mechanism(s). A continuous absorption from the supernatant of produced IgG increases the level of cellular IgG production, and hence increases production yield.

In another embodiment, the invention therefore provides a method for producing an antibody in a cell comprising culturing said cell and (semi-continuously) harvesting antibody from culture medium of said culture. In a preferred embodiment, the concentration of antibody in said culture medium is maintained at a level lower than functionally saturating levels of FcRn mediated recycling by means of said continuous harvesting. A skilled person can easily establish a saturation level of a cell by determining the amount of secreted antibody (IgG) in time and by plotting these two variables. If the amount of secreted antibody reaches a plateau value, the saturation level is determined. This is for example determined for a cell comprising natural FcRn as well as a cell (of the same kind) which has been provided with at least one additional copy of FcRn. Upon establishing the saturating levels, the above-described method is performed such that the saturating level is not reached. As a consequence, the amount of produced antibody is increased.

The efficiency of such a method is for example determined by comparing *in vitro* production of IgG in the presence of IgG-binding Sepharose-Protein A beads (Amersham Biosciences, diameter 45-165 µm) trapped behind a microporous membrane (Transwell-system, Corning, filters of 0,1-12 µM) which easily allows for penetration of secreted IgG (12x16 nm according to crystallographic and electron microscopic data ¹⁹) through the filters where it will be retained by Protein A. In this way, we interfere with (a) negative feedback mechanism(s) by circumventing 'FcRn saturation' within IgG-producing cells.

This part of the invention can optionally be combined with the earlier described invention, i.e. the invention further provides a method for producing an antibody or a functional part, derivative and/or analogue thereof in a cell, comprising providing a cell that is capable of producing said antibody with FcRn receptor protein and culturing said cell to allow for production of said antibody, wherein antibody is (semi)-continuously harvested from culture medium of said culture. Preferably, the concentration of antibody in said culture medium is maintained at a level lower than functional saturating levels of FcRn mediated recycling by means of said continuous harvesting. This is for example accomplished by using a Protein G/Protein A-sepharose filtering device. Now that the invention discloses that FcRn plays a role in the secretion of antibodies, other systems suitable for removing produced antibodies from a culture can be easily selected by the skilled person.

In yet another embodiment, the invention provides an antibody preparation obtainable by a method according to the invention.

In yet another embodiment the invention provides a pharmaceutical composition comprising such an antibody preparation.

Moreover, an antibody preparation as described above is extremely useful in the treatment of diseases such as cancer or inflammatory or infectious diseases or immune deficiencies, auto immunity or immune regulation. The invention thus also provides use of an antibody preparation according to the invention in the preparation of a medicament for the treatment of, for example, cancer, rheumatoid arthritis, Crohn's disease, asthma, psoriasis, viral respiration disease or multiple sclerosis. Examples of cancers that can be treated are non-Hodgkin's lymphoma, breast cancer, acute myleoid leukemia, chronic lymphocytic leukemia or colorectal cancer.

Now that we have shown that FcRn plays a role in the secretion of antibodies this finding can also be used to increase in vivo antibody production and hence the invention further provides a method for upregulating antibody levels in the blood of a subject comprising upregulating FcRn receptor expression in cells of said subject.

A method according to the invention is not limited to the production of antibodies but can also be used to produce a fusion protein that comprises a protein of interest and an Fc fragment. The invention thus provides a method for improving production of a protein in a cell comprising, providing said cell with a fusion of said protein and an Fc-fragment and providing said cell with FcRn receptor protein, and culturing said cell to allow for production of protein-Fc fusions products. Preferably, said Fc-fragment is derived from IgG. As already described above, a useful functional part of such an Fc-fragment is the CH2-CH3-interface.

The fusion protein is preferably provided as a nucleic acid to a cell that has optionally been provided with an FcRn receptor protein (or a functional part, derivative and/or analogue thereof). After production of said fusion product, the FcRn receptor protein will facilitate secretion of the fusion product to the culture medium and hence said fusion can easily be harvested and purified. In a preferred embodiment a protein of interest and Fc are separated by a linker. For some application it is preferable that the said linker can be cleaved with an enzyme and even more preferably the cleavage by said enzyme is such that no additional amino acid residues are attached to the protein of interest.

Examples of fusions are (synthetic) proteins of interest and the Fc of IgG or chimerics of IgA and the Fc of IgG and so on (e.g fusion of CD40 and IgG Fc ²⁰, fusion of Tumor necrosis factor Receptor (TNF-R) and Fc ²¹). In a preferred embodiment, the protein of interest is a therapeutic molecule.

The invention furthermore provides the use of FcRn receptor protein (or a functional part, derivative and/or analogue thereof) for providing (or increasing) expression of an antibody (or a functional part, derivative and/or analogue thereof) in a cell.

FcRn receptor protein (or a functional part, derivative and/or analogue thereof) may be provided as a protein or as a nucleic acid encoding said receptor protein.

If the cell in question does already contain the information for producing the desired antibody (or a functional part, derivative and/or analogue thereof), a nucleic acid encoding said antibody need not be provided. In case the cell does not contain said information, said cell is further provided with a nucleic acid encoding said antibody.

In yet another embodiment, the invention provides a method for improving production of a protein in a cell comprising, providing said cell with a fusion of said protein and albumin and providing said cell with FcRn receptor protein, and culturing said cell to allow for production of protein-albumin fusions products. As already discussed, albumin also interacts with FcRn receptor protein. In yet another embodiment, the invention further provides a method for improving production of a protein in a cell comprising, providing said cell with a fusion of said protein (or molecule) and a molecule combining a part of IgG and a part of albumin or a fusion of said protein (or molecule) with a synthetic molecule capable of binding to FcRn.

In another embodiment, the invention provides a method for improving production of albumin in a cell comprising, providing said cell with albumin and providing said cell with FcRn receptor protein, and culturing said cell to allow for production of albumin.

The invention will be explained in more detail in the following description, which is not limiting the invention.

### Materials and methods

*FcRn expression.* PMN were isolated as described in ²², NK cells, T cells, B cells and monocytes were isolated from the mononuclear fraction with a MoFlo cell sorter (Cytomation, Fort Collins, CO) using PE-labeled CD56 MAb (CLB, Amsterdam, The Netherlands), FITC-labeled anti CD3 (CLB), CD19-FITC (CLB), CD20-PE (CLB) and Biotinylated CD14 MAb (Sigma, St. Louis, MO) together with Streptavidin-APC (Molecular Probes, Leiden, the Netherlands). The different cell population were isolated using combination of these antibodies (CD14-, CD19+, CD20+ for B-cells, CD14-, CD3+ for T-cells, CD14-, CD56+ for NK cells, CD14+, CD56- for monocytes). RNA was isolated from cell populations using RNAzol (Campro Scientific, Veenendaal, The Netherlands) according to the manufacturer's instructions for mRNA quantification by real time quantitative RT-PCR analysis (Taqman), using the forward 5' CTCAGGGTGGAGCTGGAATC 3', reverse 5' CTCCACGAAGGGAGATCCAA 3', and the probe 5' ATCGTCATCGGTGTCTTGCTACTCACGG 3' primer (passes intron / exon boundaries). Arbitrary values were obtained by calculating FcRn expression levels as a ratio of ABL (Abelson) gene expression. Isolated primary cells were also stained with antibodies (see below) for confocal or FACS analyses. Confocal phagocytosis experiments were carried out as described for FACS experiments in 50 µl volumes on poly-L-lysine coated superfrost (Sigma) microscopy slides. At the end of experiments, cells were fixed with paraformaldehyde (3.7%), washed with saponin buffer (PBS with 0.5% saponin and 1% Bovine serum albumin) and incubated for 60 min at room temperature with either mouse IgG1 MAC-1 (CR3) or rabbit anti-FcRn antiserum, followed by appropriate conjugates (see below). All detection antibodies and conjugates were diluted in Saponin buffer to allow for intracellular staining. After staining, the microscopy slides were treated with ProlongGold (Molecular Probes) according to the manufacturer's recommendations, and samples were visualized by confocal microscopy as above. Samples were washed twice between incubations with PBS.

*Recombinant antibodies.* The generation and functional characteristization *in vitro* and *in vivo* of human anti-pneumococcal serotype 6A/B GDob1 antibodies and recombinant anti-meningococcal PorA antibodies used in this study have been detailed before in ^{22,23}. The mutated IgG1 GDob1 variants was generated by mutating the γ1 heavy chain using a Quickchange Site-directed-mutagenesis kit (Stratagene, CA) according to the manufacturer's guidelines, using CH3-specific primers incorporating the specific mutations. After verification by sequencing (ABI 373 Stretch automated sequencing machine, Applied Biosystems, Foster City, CA) of the correct incorporation of the mutated bases, resulting in the corresponding amino acid change from a Histidine to an Alanine in position 435, the heavy chain was transfected together with the corresponding light chain, produced and purified, as described in ^{22,23}. Antibody amounts were quantified by a sandwich ELISA, capturing the recombinant antibodies by a rabbit anti-kappa antiserum, using alkaline phosphatase-conjugated rabbit anti-IgG (Fc specific) antisera for detection as described in ²², and by antigen-specific ELISA, as detailed in ²³.

*Detection antibodies.* Protein G isolated mouse IgG1 directed against the α-chain of FcRn (MAb 1G3) was obtained from ATCC (Manassas, VA) ²⁴. Mouse IgM MAb 22H4C11 was raised by immunizing balb/c mice with a peptide stretch composing the FcRn α-chain intracellular tail. Rabbit antiserum against human FcRn was a generous gift of Dr. Neil Simister (Brandeis University, Waltham, MA). Mouse IgG1 was detected in FACS experiments using Fitc-labeled goat F(ab')2-fragments of anti-mouse IgG (Protos, Burlingame, CA). Rabbit antiserum was detected with Biotin-labeled swine anti-rabbit IgG F(ab')₂-fragments (DAKO, Glostrup, Denmark), followed by Streptavidin-Alexa555 (Molecular Probes, Leiden, The Netherlands).

*FcRn induced expression.* A single clone of BHK cells transfected with IgG1 (pNUT, metallothionin promoter, DHFR/Methatrexate selection marker) ²² and stably expressing IgG1 directed against Porin A of *Neisseria meningitidis,* was transfected with human FcRn (pcDNA3.1, CMV promoter / zeocin selection marker) and single clones expressing both IgG1 and FcR were selected by zeocin tolerance and limiting dilution. FcRn expression was determined with the monoclonal 1G3 by FACS analyses, and IgG1 levels in supernatants was measured by PorA specific ELISA (as described above, "Recombinant antibodies", after seeding identical amounts of cells into T-culture flasks and harvesting supernatants on day 3.

*Negative feedback influence of IgG on IgG production:* 293 cells were transfected with GDob1 IgG (described above) with or without exogenously added IgG polyclonal IgG (IVIG, CLB). Samples of supernatants were taken at the times indicated, and anti-pneumococcal 6B antibodies were measured by antigen-specific ELISA as described in ²³.

*Statistical analyses. Pearson* Correlation using GraphPad Prism version 4.00 for Windows (GraphPad Software, San Diego CA, USA, http://www.graphpad.com). Significance was set at p<0.05.

### Results

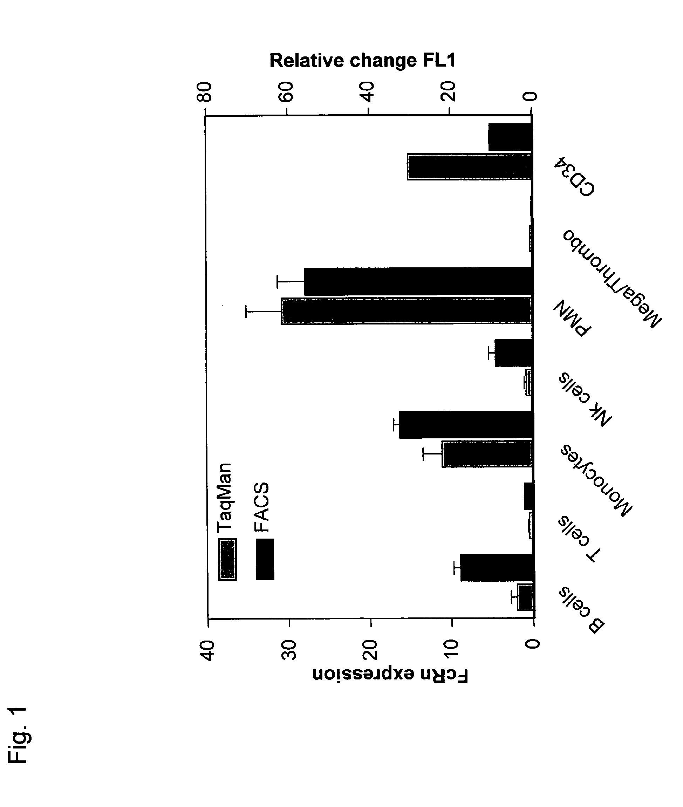

*Expression of FcRn on immune cells.* PMN were isolated as described in ²², and NK cells, T cells, B cells and monocytes sorted by FACS from the mononuclear fraction. FcRn expression was then determined by real-time quantitative PCR, or stained with an FcRn-specific monoclonal antibody. FcRn expression was most prominent on PMN, monocytes, B cells and on lymphohematopoietic stem and progenitor cells (CD34+), but low or absent on NK and T cells (Figure 1).

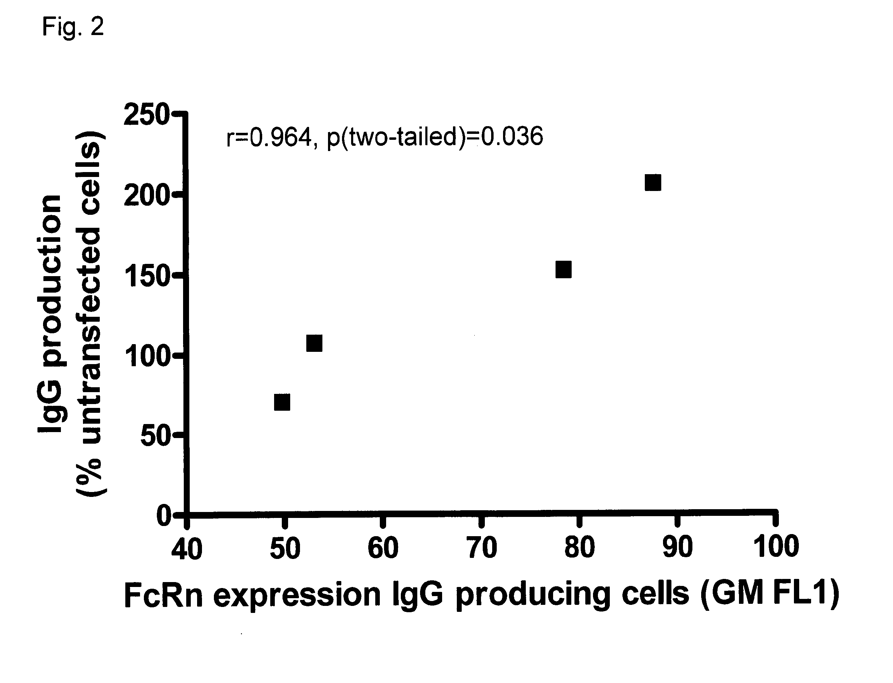

*FcRn enhances production of IgG in antibody-producing cells.* To test whether FcRn can influence production of IgG1 by IgG-producing cells, we transfected human FcRn to a stable IgG1-expressing BHK clone. Clones were selected after FcRn transfection, and expression of FcRn was compared between clones by FACS analyses. Production of IgG1 was also studied in supernatants. We observed high FcRn levels correlate significantly with increased levels of IgG1 production (Figure 2). Thus, introduction of high human FcRn levels into human-IgG producing-cells readily increased antibody production.

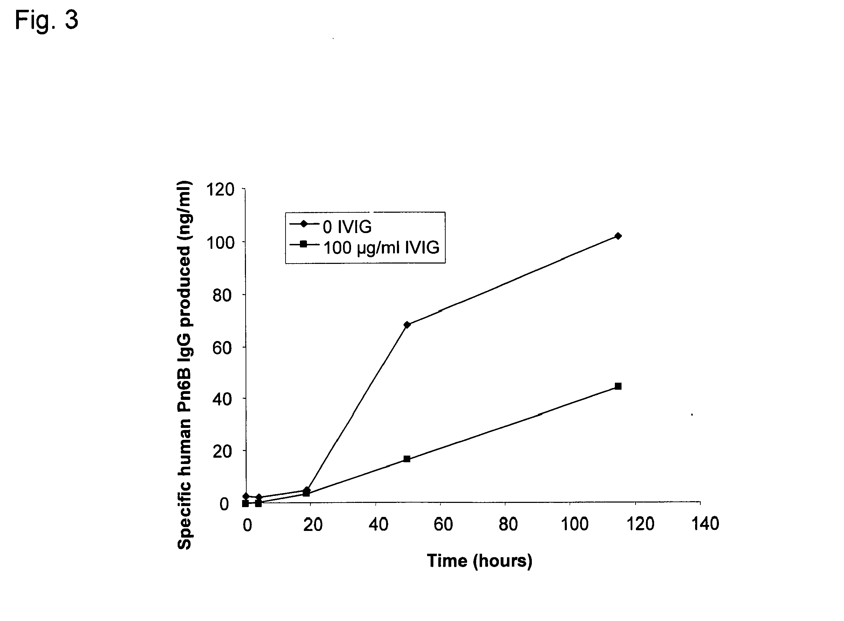

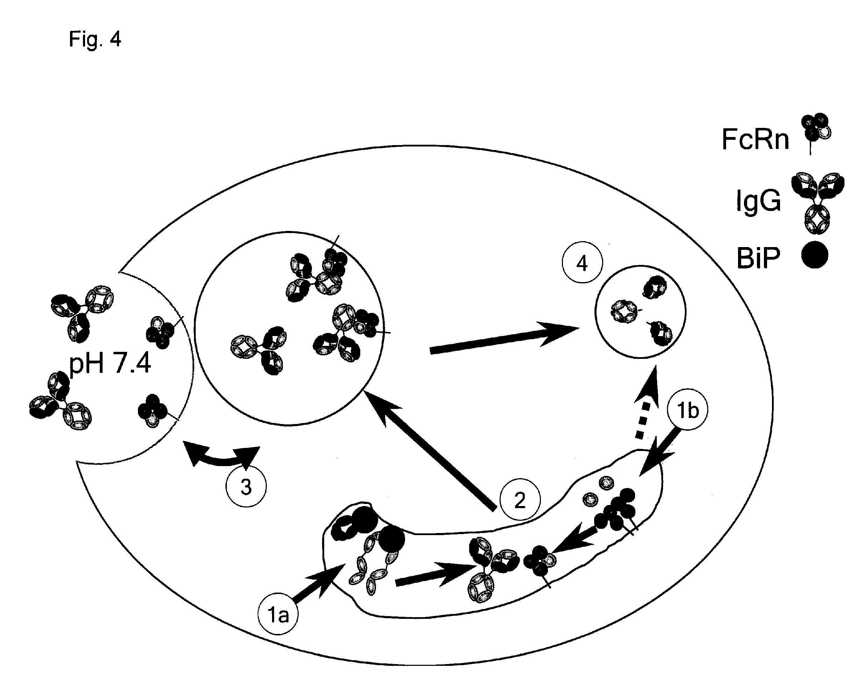

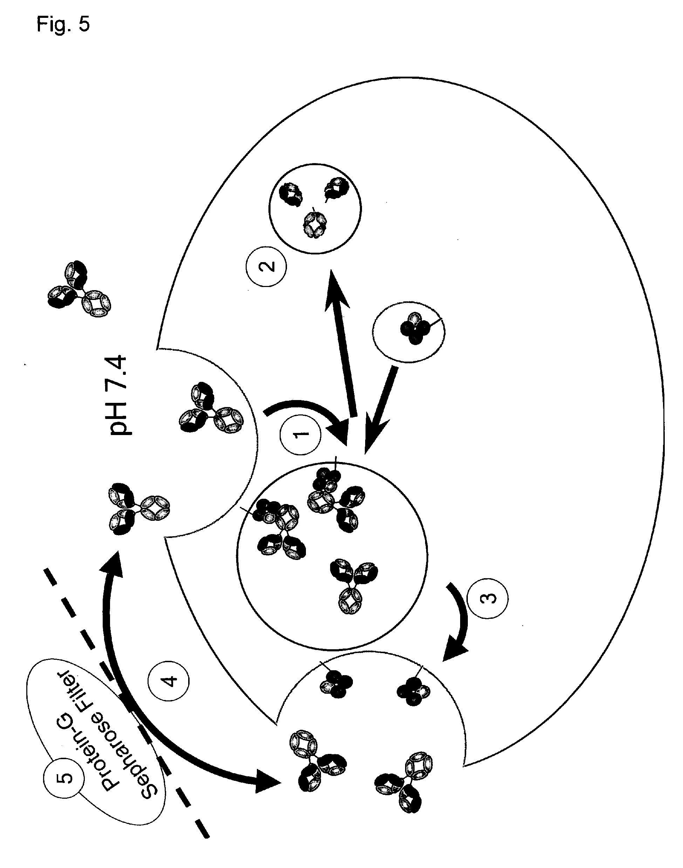

*IgG production may be regulated by a negative-feedback mechanism.* FcRn-mediated IgG recycling is saturable *in vivo* ^{4,12}, thus not only secretion of IgG by IgG-producing cells *in vitro* may be dependent on FcRn, but also its recycling. To address this experimentally, we performed competition studies, in which we cultured equal numbers of human 293 cells (which express endogenous FcRn) with no, or increasing amounts of exogenous polyclonal human IgG. Exogenously added IgG was shown to inhibit IgG secretion from these cells (Figure 3), in accordance with a saturable nature of IgG-recycling by FcRn.

### Description of figures

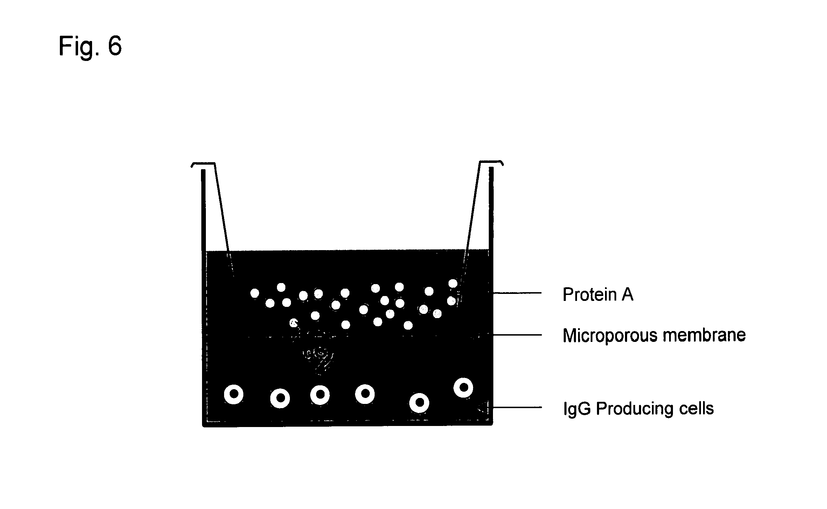

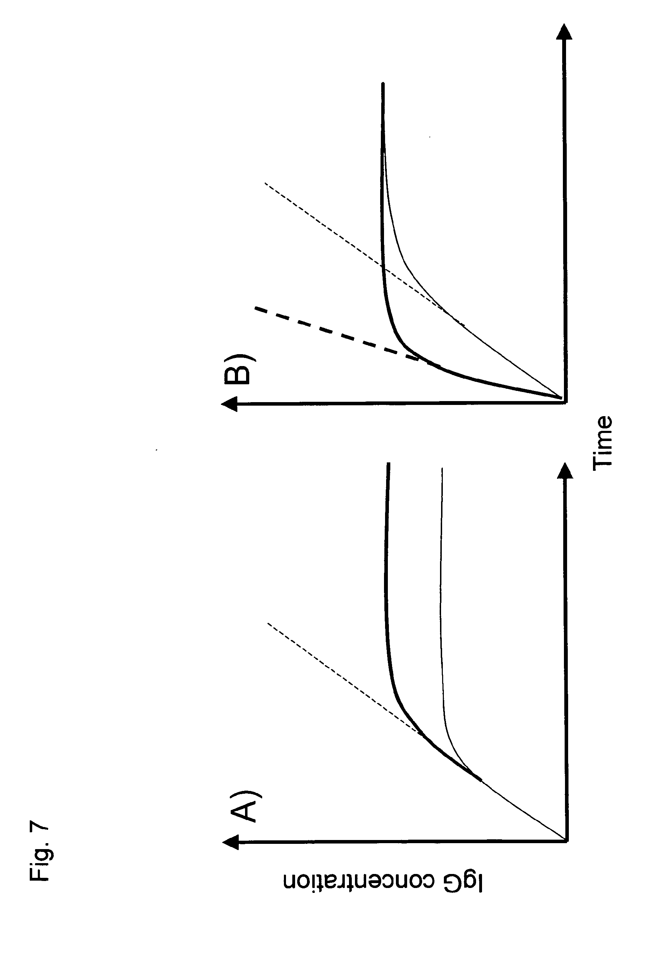

**Figure 1**
   Expression of FcRn in human blood cells. FcRn mRNA and protein levels was measured by real-time quantitative RT-PCR, and flow cytometry. FcRn expression in cells was detected by MAb 22H4C11.
**Figure 2**
   FcRn expression correlates with increased IgG1 production *in vitro.* BHK cells were transfected with IgG1 (pNUT, metallothionin promoter) ²² directed against Porin A of *Neisseria meningitidis.* A single clone, stably expressing IgG1 was subsequently transfected with human FcRn (pcDNA3.1, CMV promoter). FcRn expression was determined with the monoclonal 1G3 by FACS analyses, and IgG1 levels in supernatants was measured by PorA specific ELISA, after seeding identical amounts of cells into T-culture flasks and harvesting supernatants on day 3. Each dot represents FcRn and IgG1 production levels of a single subclone. IgG production levels are presented as % production of original mock-transfected clone (4,9 µg/ml = 100%). Correlation coefficient, r=0.964, p(two-tailed)=0.036.
**Figure 3**
   Exogenously added IgG inhibits the production of IgG by 293 cells, indicating that FcRn-mediated IgG-recycling in these cells is saturable. 293 cells producing human IgG1 directed against *S. pneumoniae* ²³, were incubated in the presence of purified poloyclonal IgG (intravenous immunoglobulin, IVIG, Sanquin products, Amsterdam). Samples of supernatants were taken at the times indicated, and anti-pneumococcal 6B antibodies were measured by antigen-specific ELISA²³ (unpublished data, n=3).
**Figure 4**
   Model for IgG production in B cells or tranfectomas. 1a) Polypeptide chains encoding for the light chains and heavy chains of IgG are translated on the endoplasmic reticulum (ER) and assembled with the aid of molecular chaperones, including BiP. Polypeptide chains encoding for the α-chain of FcRn, and the β2M are also translated on the ER and assembled through oligomeric and dimeric transition phase, after which it assembles with β2M. FcRn can bind IgG both before and after assembly with β2M ^{25,26}. 2) When fully assembled, FcRn is transported, with or without cargo (IgG), through sorting endosomes or lysosomes, 3) from where FcRn can transport IgG to the surface where it is released at pH>7.4. 4) When FcRn is saturated, no more IgG can be bound, and is becomes a subject to degradation after further maturation of the lysosomes ^{27,28}. Without efficient transport out of ER, the same fate awaits newly synthesized IgG ^{29,30} and FcRn ^{25,26,31.}
**Figure 5**
   Fluid-phase IgG is subject to fluid-phase pinocytosis and subsequent degradation. 1) Cells take up IgG by pinocytosis, which then becomes available for binding to FcRn at lysosomal pH (≤ 6.5). 2) Unbound IgG (no FcRn or FcRn is saturated) degraded in lysosomes. 3) IgG bound to FcRn is recycled back to the surface where it is released into the serosa/supernatant at neutral pH. 4) Recycled IgG can be pinocytosed by cells (other or the same cell). 5) The recycling process, and subsequent degradation, are overcome by using a Protein G / Protein A-sepharose filtering device.
**Figure 6**
   IgG "capturing device". Set-up to investigate the feasibility to capture IgG secreted by IgG-producing cells *in vitro* by Protein A through a microporous membrane allowing for passage of IgG, but neither of cells or Protein A sepharose beads. IgG in the supernatants is therefore less likely to reach saturating levels within the cells, and less likely to be taken up by pinocytosis by IgG-producing cells (where it may otherwise be a subject to degradation). See text and Figure 6 for details.
**Figure 7**
   Expected kinetics of IgG secretion with or without FcRn overexpression of if A) FcRn is only involved in IgG recycling and not in secretion B) if FcRn is only involved secretion. Wild-type cells: solid line, Cells-overexpressing FcRn: bold lines. Dotted lines: Expected production with systems involving IgG capturing device (See Figure 6 above).

### References

1 van Dijk, M.A. and Vidarsson, G. (2002) Monoclonal antibody-based Pharmaceuticals. In Pharmaceutical Biotechnology: An Introduction for Pharmacists and Pharmaceutical Scientists (Crommelin, D.J.A. and Sindelar, R.D., eds.), pp. 283-297, Taylor & Francis
2 van Dijk, M.A. and van de Winkel, J.G. (2001) Human antibodies as next generation therapeutics. Curr Opin Chem Biol 5 (4), 368-374.
3 Kohler, G. and Milstein, C. (1975) Continuous cultures of fused cells secreting antibody of predefined specificity. Nature 256 (5517), 495-497
4 Lobo, E.D. et al. (2004) Antibody pharmacokinetics and pharmacodynamics. J Pharm Sci 93 (11), 2645-2668
5 West, A.P., Jr. and Bjorkman, P.J. (2000) Crystal structure and immunoglobulin G binding properties of the human major histocompatibility complex-related Fc receptor(,). Biochemistry 39 (32), 9698-9708
6 Shields, R.L. et al. (2001) High resolution mapping of the binding site on human IgG1 for Fc gamma RI, Fc gamma RII, Fc gamma RIII, and FcRn and design of IgG1 variants with improved binding to the Fc gamma R. J Biol Chem 276 (9), 6591-6604.
7 Martin, W.L. et al. (2001) Crystal structure at 2.8 A of an FcRn/heterodimeric Fc complex: mechanism of pH-dependent binding. Mol Cell 7 (4), 867-877.
8 Simister, N.E. and Mostov, K.E. (1989) An Fc receptor structurally related to MHC class I antigens. Nature 337 (6203), 184-187.
9 Yoshida, M. et al. (2004) Human neonatal Fc receptor mediates transport of IgG into luminal secretions for delivery of antigens to mucosal dendritic cells. Immunity 20 (6), 769-783
10 Story, C.M. et al. (1994) A major histocompatibility complex class I-like Fc receptor cloned from human placenta: possible role in transfer of immunoglobulin G from mother to fetus. J Exp Med 180 (6), 2377-2381.
11 Roopenian, D.C. et al. (2003) The MHC Class I-Like IgG Receptor Controls Perinatal IgG Transport, IgG Homeostasis, and Fate of IgG-Fc-Coupled Drugs. J Immunol 170 (7), 3528-3533.
12 Junghans, R.P. and Anderson, C.L. (1996) The protection receptor for IgG catabolism is the beta2- microglobulin-containing neonatal intestinal transport receptor. Proc Natl Acad Sci U S A 93 (11), 5512-5516
13 Ghetie, V. et al. (1996) Abnormally short serum half-lives of IgG in beta 2-microglobulin-deficient mice. Eur J Immunol 26 (3), 690-696.
14 Israel, E.J. et al. (1996) Increased clearance of IgG in mice that lack beta 2-microglobulin: possible protective role of FcRn. Immunology 89 (4), 573-578.
15 Vidarsson, G. et al. (2005) The MHC class-I homolog, FcRn, facilitates IgG-mediated phagocytosis. Submitted
16 Chaudhury, C. et al. (2003) The major histocompatibility complex-related Fc receptor for IgG (FcRn) binds albumin and prolongs its lifespan. J Exp Med 197 (3), 315-322.
17 Burmeister, W.P. et al. (1994) Crystal structure at 2.2 A resolution of the MHC-related neonatal Fc receptor. Nature 372 (6504), 336-343.
18 Burmeister, W.P. et al. (1994) Crystal structure of the complex of rat neonatal Fc receptor with Fc. Nature 372 (6504), 379-383
19 Saphire, E.O. et al. (2001) Crystal structure of a neutralizing human IGG against HIV-1: a template for vaccine design. Science 293 (5532), 1155-1159
20 Noelle, R.J. et al. (1992) A 39-kDa protein on activated helper T cells binds CD40 and transduces the signal for cognate activation of B cells. Proc Natl Acad Sci U S A 89 (14), 6550-6554
21 Sandalon, Z. et al. (2004) Secretion of a TNFR:Fc fusion protein following pulmonary administration of pseudotyped adeno-associated virus vectors. J Virol 78 (22), 12355-12365
22 Vidarsson, G. et al. (2001) Activity of Human IgG and IgA Subclasses in Immune Defense Against Neisseria meningitidis Serogroup B. J Immunol 166 (10), 6250-6256.
23 Saeland, E. et al. (2003) Central Role of Complement in Passive Protection by Human IgG1 and IgG2 Anti-pneumococcal Antibodies in Mice. J Immunol 170 (12), 6158-6164.
24 Raghavan, M. et al. (1994) Investigation of the interaction between the class I MHC-related Fc receptor and its immunoglobulin G ligand. Immunity 1 (4), 303-315
25 Praetor, A. and Hunziker, W. (2002) beta(2)-Microglobulin is important for cell surface expression and pH-dependent IgG binding of human FcRn. J Cell Sci 115 (Pt 11), 2389-2397
26 Zhu, X. et al. (2002) The heavy chain of neonatal Fc receptor for IgG is sequestered in endoplasmic reticulum by forming oligomers in the absence of beta2-microglobulin association. Biochem J 367 (Pt 3), 703-714
27 Ober, R.J. et al. (2004) Exocytosis of IgG as mediated by the receptor, FcRn: an analysis at the single-molecule level. Proc Natl Acad Sci U S A 101 (30), 11076-11081
28 Ober, R.J. et al. (2004) Visualizing the site and dynamics of IgG salvage by the MHC class I-related receptor, FcRn. J Immunol 172 (4), 2021-2029
29 Leitzgen, K. et al. (1997) Assembly of immunoglobulin light chains as a prerequisite for secretion. A model for oligomerization-dependent subunit folding. J Biol Chem 272 (5), 3117-3123
30 Wiens, G.D. et al. (2001) Mutation of a single conserved residue in VH complementarity-determining region 2 results in a severe Ig secretion defect. J Immunol 167 (4), 2179-2186
31 Praetor, A. et al. (2002) Membrane-anchored human FcRn can oligomerize in the absence of IgG. J Mol Biol 321 (2), 277-284

### Tables

**Table 1: Therapeutic antibodies that have been approved by the FDA**

| Company Name | Name of Product | Indications | Year of Approval | Antibody Type |
|---|---|---|---|---|
| Ortho Biotech | **Orthoclone-OKT®** | Organ Transplant Rejection | 1986 | M |
| J&J/Eli Lilly | **ReoPro®** | Acute Cardiac Conditions | 1994 | C |
| BiogenIdec/Genentech/Roche | **Rituxan®** | Non-Hodgkin's Lymphoma | 1997 | C |
| PDLI | **Zenapax®** | Acute Transplant Rejection | 1997 | H |
| MedImmune/Abbott | **Synagis®** | Viral Respiratory Disease | 1998 | H |
| J & J | **Remicade®** | Crohn's, Rheumatoid Arthritis | 1998 | C |
| Novartis | **Simulect®** | Acute Transplant Rejection | 1998 | C |
| Genentech/Roche | **Herceptin®** | Breast Cancer | 1998 | H |
| Wyeth | **Mylotarg^{™}** | Acute Myleoid Leukemia | 2000 | H |
| Schering /ILEX Oncology | **Campath®** | Chronic Lymphocytic Leukemia | 2001 | H |
| Abbott/CAT | **Humira^{™}** | Rheumatoid Arthritis | 2002 | PD |
| Biogen Idec/Genentech | **Zevalin** | Non-Hodgkin's Lymphoma | 2002 | M |
| Novartis/Genentech/Tanox | **Xolair®** | Asthma | 2003 | H |
| Genentech/Xoma | **Raptiva^{™}** | Psoriasis | 2003 | H |
| Corixa/GlaxoSmithKline | **Bexxar®** | Non-Hodgkin's Lymphoma | 2003 | M |
| Genentech/Roche | **Avastin**® | Colorectal Cancer | 2004 | H |
| BMS/ImClone Systems | **Erbitux^{™}** | Colorectal Cancer | 2004 | C |
| Biogen Idec | Tysabri | Multiple Sclerosis | 2004 | H |

| | | | | |
|---|---|---|---|---|
| H: Humanized, M: Mouse, C: Chimeric, PD: Phage display (human) | | | | |

## Claims

1. A method for producing an antibody or a functional part, derivative and/or analogue thereof in a cell, comprising providing a cell that is capable of producing said antibody with FcRn receptor protein and culturing said cell to allow for production of said antibody.

2. A method according to claim 1, wherein said cell is provided with a nucleic acid encoding said FcRn receptor protein.

3. A method according to claim 2, comprising over-producing said FcRn receptor protein in said cell.

4. A method according to any one of claims 1 to 3, wherein said antibody is derived from a first species and said cell is derived from a different species.

5. A method according to any one of claims 1 to 4, wherein at least the α-chain of FcRn receptor protein is provided or over-produced.

6. A method according to claim 5, further comprises providing or over-producing β2-microglobulin.

7. A method according to claim 5 or 6 wherein said α-chain and/or said β2-microglobulin is derived from the same species.

8. A method according to claim 7, wherein said α-chain and/or said β2-microglobulin is derived from the same species that said cell is derived from.

9. A method according to claim 5, wherein antibody produced by said cell is derived from the same species as said FcRn receptor α-chain, or derived from a functional analogue of said species.

10. A method according to claim 9, wherein said functional analogues species is derived from the same genus.

11. A method according to any one of claims 1-10, wherein said antibody is derived from a human.

12. A method according to any one of claims 1-11, wherein said cell is derived from a rodent, preferably a Chinese hamster or a mouse.

13. A method according to any one of claims 1 to 12, wherein said antibody is a monoclonal antibody.

14. A method according to any one of claims 1 to 13, wherein said cell is a hybridoma or a transfectoma.

15. A method according to claim 14, wherein said transfectoma is a cell that has been provided with a nucleic acid encoding said antibody to obtain a cell that is capable of producing said antibody.

16. A method for producing an antibody in a cell comprising culturing said cell and (semi-continuously) harvesting antibody from culture medium of said culture.

17. A method according to claim 16, wherein the concentration of antibody in said culture medium is maintained at a level lower than functional saturating levels of FcRn mediated recycling.

18. A method according to claim 17, wherein the concentration of antibody in said culture medium is maintained at a level lower than functional saturating levels of FcRn mediated recycling by means of said continuous harvesting.

19. A method according to any one of claims 1-15, wherein antibody is (semi)-continuously harvested from culture medium of said culture.

20. A method according to claim 19, wherein the concentration of antibody in said culture medium as maintained at a level lower than functional saturating levels of FcRn mediated recycling by means of said continuous harvesting.

21. A method according to claim 20, wherein the concentration of antibody in said culture medium is maintained at a level lower than functional saturating levels of FcRn mediated recycling by means of said continuous harvesting.

22. An antibody preparation obtainable by a method according to any one of claims 1-15 or 19-21.

23. A pharmaceutical composition comprising an antibody preparation according to claim 22.

24. Use of an antibody preparation according to claim 22 in the preparation of a medicament for the treatment of cancer, inflammatory, infectious diseases, immune deficiencies, auto immunity or immune regulation

25. Use according to claim 24, wherein said cancer is non-Hodgkin's lymphoma, breast cancer, acute myleoid leukemia, chronic lymphocytic leukemia or colorectal cancer.

26. A method for upregulating antibody levels in the blood of a subject comprising upregulating FcRn receptor expression in cells of said subject.

27. A method for improving production of a protein in a cell comprising, providing said cell with a fusion of said protein and an Fc-fragment and providing said cell with FcRn receptor protein, and culturing said cell to allow for production of protein-Fc fusions products.

28. A method for improving production of a protein in a cell comprising, providing said cell with a fusion of said protein and albumin and providing said cell with FcRn receptor protein, and culturing said cell to allow for production of protein-albumin fusions products.

29. A method for improving production of albumin in a cell comprising, providing a cell capable of producing albumin with FcRn receptor protein, and culturing said cell to allow for production of albumin.
